# EUROPEAN PATENT SPECIFICATION

(11) **EP 3 454 062 B1**
(45) Date of publication and mention of the grant of the patent: **07.10.2020**
(21) Application number: 18185430.8
(22) Date of filing: 25.07.2018
(51) Int. Cl.: G01N 33/49

(54) **BLOOD FILTRATION UNIT**
BLUTFILTRATIONSEINHEIT
UNITÉ DE FILTRATION DU SANG

(30) Priority: 08.09.2017 JP 2017173351
(43) Date of publication of application: 13.03.2019
(73) Proprietor: Fujifilm Corporation, Minato-ku Tokyo 106-8620 (JP)
(72) Inventor: TADOKORO, Shintaro, Kanagawa, Kanagawa 258-8538 (JP)
(74) Representative: Klunker IP Patentanwälte PartG mbB

(56) References cited:
- JP-A- 2000 009 726
- JP-A- 2000 081 432
- US-B1- 6 280 621

## Description

### BACKGROUND

### Technical Field

The present invention relates to a blood filtration unit.

### Related Art

JP1999-295298A (JP-H11-295298A) and JP2000-81432A disclose a blood filtration unit in which blood is filtered by a filtration portion housing a blood filtration material and the filtered filtrate (plasma or serum) is stored in a tubular filtrate receiving tank (storage portion). In particular, the blood filtration unit disclosed in JP1999-295298A (JP-H11-295298A) has a configuration capable of filtering blood without requiring a separate decompression device, by openably sealing an opening of a nozzle for sucking blood and a filtrate outlet (outflow port) and setting the interior of the unit to be in a decompression condition. In addition, the blood filtration unit disclosed in JP2000-81432A has a configuration in which liquid retaining properties in the vicinity of the filtrate outlet are reduced by removing a facing surface provided in a filtrate outlet or setting the length of a lower side of the filtrate outlet to be longer than or equal to 0.8 mm.

### SUMMARY

However, in the blood filtration unit disclosed in JP1999-295298A (JP-H11-295298A) and JP2000-81432A, a structure for suppressing wet-spreading (wetting) of the filtrate flowing out from the outflow port has not been studied. For this reason, the filtrate flowing out from the outflow port is wet-spread, and therefore, there is a possibility that the filtrate may flow outside of a storage portion along an opening end surface of the storage portion.

US 6 280 621 B1 discloses a blood filtration unit according to the preamble of claim 1. The outflow port of the outflow portion is formed such that it opens directly into the storage portion while extending over a slanted surface formed in the opening end surface of the storage portion, while on both sides of the outflow port screens are provided for guiding filtrate and avoiding scattering thereof.

In view of the above-described facts, an object of the present invention is to obtain a blood filtration unit capable of suppressing wet-spreading of a filtrate flowing out from an outflow port.

In order to solve the above-described problems, there is provided a blood filtration unit according to a first aspect of the present invention comprising: a filtration portion in which a blood filtration material is housed; a blood inflow portion through which blood flows into the filtration portion; a storage portion which has a tubular shape with one end opened and in which a filtrate filtered by the filtration portion is stored; a filtrate flow path portion which is formed along the outer periphery of the storage portion and includes a flow path through which the filtrate flows; an outflow portion in which an outflow port is formed between a pair of wall portions protruding beyond the one end of the storage portion from an end portion of the filtrate flow path portion along an opening end surface of the one end of the storage portion; and a guide portion which is formed on the opening end surface of the storage portion and includes at least one of a protruding portion or a groove portion extending from the pair of wall portions toward the opening.

According to the present invention, the blood filtration unit has the features of claim 1. Accordingly, the blood flowing from the blood inflow portion into the filtration portion is filtered by the blood filtration material, and the filtrate flows from the outflow port to the storage portion through the flow path of the filtrate flow path portion and is stored in the storage portion.

In addition, the guide portion is formed on the opening end surface of the storage portion and includes at least one of the protruding portion or the groove portion extending from the pair of wall portions toward the opening of the storage portion. Accordingly, although liquid droplets of the filtrate flowing out from the outflow port tend to wet-spread while coming into contact with the opening end surface, it is possible to suppress the wet-spreading of the filtrate using the liquid droplets coming into contact with the guide portion.

In a blood filtration unit according to a second aspect of the present invention, the guide portion in the blood filtration unit according to the first aspect includes the protruding portion.

According to the blood filtration unit of the second aspect of the present invention, it is possible to suppress the wet-spreading of the filtrate using liquid droplets of the filtrate which come into contact with the protruding portion.

In a blood filtration unit according to a third aspect of the present invention, the protruding portion in the blood filtration unit according to the second aspect has a triangular cross section cut in a direction orthogonal to an extending direction.

According to the blood filtration unit of the third aspect of the present invention, by forming the cross section of the protruding portion in a triangular shape, it is possible to effectively suppress the wet-spreading since the liquid droplets of the filtrate hardly go around (climb over) the protruding portion compared to a configuration in which an apex portion of the protruding portion is formed into a curved surface.

In a blood filtration unit according to a fourth aspect of the present invention, the protruding portions in the blood filtration unit according to the second or third aspect extend parallel to each other from one of the wall portions and the other wall portion while interposing the outflow port therebetween.

According to the blood filtration unit of the fourth aspect of the present invention, it is possible to make the liquid droplets of the filtrate reliably come into contact with the protruding portions on both sides of the outflow port and to effectively suppress the wet-spreading of the filtrate.

In a blood filtration unit according to a fifth aspect of the present invention, a height of the protruding portion with respect to the opening end surface in the blood filtration units according to any one of the second to fourth aspects is 1 µm to 2 mm.

According to the blood filtration unit of the fifth aspect of the present invention, it is possible to effectively suppress the wet-spreading of the filtrate by setting the height of the protruding portion with respect to the opening end surface to the above-described range. That is, in a case where the height of the protruding portion with respect to the opening end surface is lower than 1 µm, the function as the protruding portion cannot be sufficiently achieved. In addition, in a case where the height of the protruding portion with respect to the opening end surface is set to be higher than 2 mm, a holding volume of liquid droplets is increased, and therefore, the filtrate easily flows to the outside of the storage portion.

In a blood filtration unit according to a sixth aspect of the present invention, a plurality of the protruding portions in the blood filtration units according to any one of the second to fifth aspects extend from one of the wall portions and the other wall portion while interposing the outflow port therebetween.

According to the blood filtration unit of the sixth aspect of the present invention, even in a case where the liquid droplets climb over one protruding portion, it is possible to suppress the wet-spreading of the liquid droplets at a second and subsequent protruding portions. That is, it is possible to suppress the wet-spreading of the filtrate effectively compared to a configuration in which one protruding portion is respectively provided in one wall portion and the other wall portion.

In a blood filtration unit according to a seventh aspect of the present invention, an interval between the protruding portions adjacent to each other on the one wall portion side and an interval between the protruding portions adjacent to each other on the other wall portion side in the blood filtration unit according to the fifth aspect are 5 µm to 1 mm.

According to the blood filtration unit of the seventh aspect of the present invention, it is possible to effectively suppress the wet-spreading of the filtrate by setting the interval of the adjacent protruding portions to the above-described range. That is, in a case where the interval (pitch) between the adjacent protruding portions is less than 5 µm, it is difficult to obtain desired dimensional accuracy. In addition, in a case where the pitch is greater than 1 mm, the wet-spreading amount until the liquid droplets come into contact with the protruding portions increases.

In a blood filtration unit according to an eighth aspect of the present invention, the guide portion in the blood filtration unit according to the first aspect includes the groove portion.

According to the blood filtration unit of the eighth aspect of the present invention, liquid droplets hardly go around a portion in which an angle variation is steep, and therefore, it is possible to suppress the wet-spreading of the filtrate since the liquid droplets of the filtrate stop at an edge of the groove portion.

In a blood filtration unit according to a ninth aspect of the present invention, the groove portion in the blood filtration unit according to the eighth aspect has a shape cut out into a rectangle as seen from a direction orthogonal to an extending direction.

According to the blood filtration unit of the ninth aspect of the present invention, it is possible to reduce an angle between the opening end surface and a groove wall and to effectively suppress the wet-spreading of the filtrate, compared to a case where the groove portion has a shape cut out into a triangle.

In a blood filtration unit according to a tenth aspect of the present invention, the groove portions in the blood filtration unit according to the eighth or ninth aspect extend parallel to each other from one of the wall portions and the other wall portion while interposing the outflow port therebetween.

According to the blood filtration unit of the tenth aspect of the present invention, it is possible to make the liquid droplets of the filtrate reliably follow edges of the groove portions on both sides of the outflow port and to effectively suppress the wet-spreading of the filtrate.

In a blood filtration unit according to an eleventh aspect of the present invention, a depth of the groove portion with respect to the opening end surface in the blood filtration units according to any one of the eighth to tenth aspects is 1 µm to 2 mm.

According to the blood filtration unit of the eleventh aspect of the present invention, it is possible to effectively suppress the wet-spreading of the filtrate by setting the depth of the groove portion with respect to the opening end surface to the above-described range. That is, in a case where the depth of the groove portion with respect to the opening end surface is shallower than 1 µm, the function as the groove portion cannot be sufficiently exerted. In addition, in a case where the depth of the groove portion with respect to the opening end surface is made deeper than 2 mm, the moldability in a case of molding performed through injection molding or the like sometimes decreases.

In a blood filtration unit according to a twelfth aspect of the present invention, a plurality of the groove portions in the blood filtration units according to any one of the eighth to eleventh aspects extend from one of the wall portions and the other wall portion while interposing the outflow port therebetween.

According to the blood filtration unit of the twelfth aspect of the present invention, even in a case where the liquid droplets pass through one groove portion, it is possible to suppress the wet-spreading of the liquid droplets at a second and subsequent groove portions. That is, it is possible to suppress the wet-spreading of the filtrate effectively compared to a configuration in which one groove portion is respectively provided in one wall portion and the other wall portion.

In a blood filtration unit according to a thirteenth aspect of the present invention, an interval between the groove portions adjacent to each other on the one wall portion side and an interval between the groove portions adjacent to each other on the other wall portion side in the blood filtration unit according to the twelfth aspect are 5 µm to 1 mm.

According to the blood filtration unit of the thirteenth aspect of the present invention, it is possible to effectively suppress the wet-spreading of the filtrate by setting the interval of the adjacent groove portions to the above-described range. That is, in a case where the interval (pitch) between the adjacent groove portions is less than 5 µm, it is difficult to obtain desired dimensional accuracy. In addition, in a case where the pitch is greater than 1 mm, the wet-spreading amount until the liquid droplets come into contact with the edge of the groove portions increases.

In a blood filtration unit according to a fourteenth aspect of the present invention, the blood filtration material in the blood filtration units according to any one of the first to thirteenth aspects includes glass fiber filter paper and a microporous film.

According to the blood filtration unit of the fourteenth aspect of the present invention, it is possible to suppress leaking out or elution of blood cell components compared to a configuration in which only one of glass fiber filter paper and a microporous film is provided.

In a blood filtration unit according to a fifteenth aspect of the present invention, the blood filtration material in the blood filtration units according to any one of the first to fourteenth aspects is formed of a material of which a void volume is 40% to 95%.

According to the blood filtration unit of the fifteenth aspect of the present invention, it is possible to effectively perform the filtration compared to a case where a blood filtration material having a void volume lower than 40% is used.

As described above, in the blood filtration unit according to the present invention, it is possible to suppress the wet-spreading of the filtrate flowing out from the outflow port.

### BRIEF DESCRIPTION OF THE DRAWINGS

Exemplary Embodiments of the present invention will be described in detail with reference to the following figures, wherein:
Fig. 1 is a longitudinal cross-sectional view of a blood filtration unit according to a first embodiment.
Fig. 2 is a plan view of a lid body constituting the blood filtration unit according to the first embodiment.
Fig. 3 is a bottom surface view of the lid body constituting the blood filtration unit according to the first embodiment.
Fig. 4 is a perspective view of a periphery of an outflow portion of the blood filtration unit according to the first embodiment.
Fig. 5 is an enlarged perspective view of protruding portions of Fig. 4.
Fig. 6 is a perspective view of the periphery of the outflow portion of the blood filtration unit according to the first embodiment which schematically shows a state in which liquid droplets of a filtrate come into contact with the protruding portions.
Fig. 7 is a perspective view of a periphery of an outflow portion of a blood filtration unit according to a comparative example.
Fig. 8 is an enlarged perspective view of groove portions of a blood filtration unit according to a second embodiment.
Fig. 9 is a perspective view, corresponding to Fig. 8, which shows a modification example of the groove portions of the blood filtration unit according to the second embodiment.

### DETAILED DESCRIPTION

### First Embodiment

Hereinafter, a first embodiment of the present invention will be described with reference to the drawings. In the drawings, the same reference numerals are given to constituent elements having the same function, and the description thereof will not be repeated as appropriate.

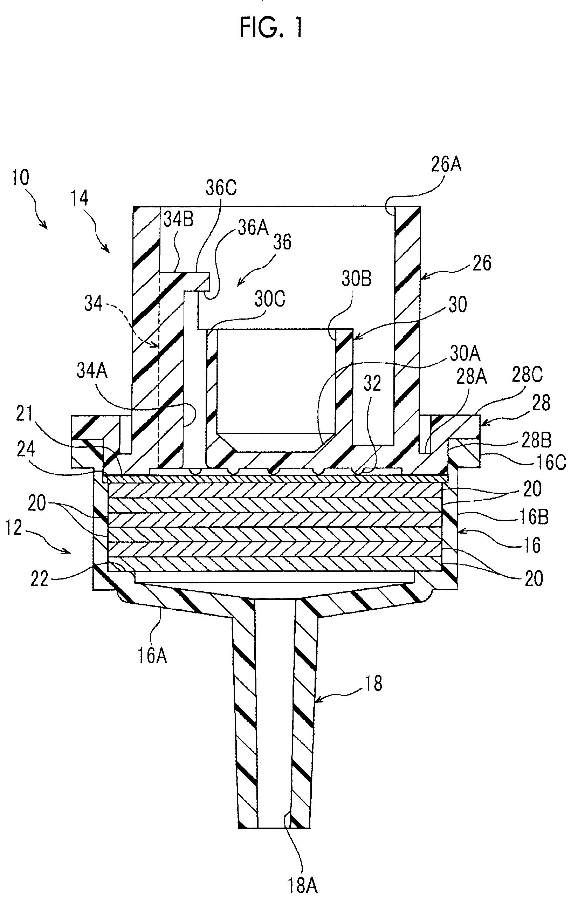

As shown in Fig. 1, the blood filtration unit 10 of the present embodiment includes a holder main body 12 and a lid body 14. In the following description, for convenience, the lid body 14 is set as an upper side as seen from the holder main body 12 in the blood filtration unit 10 and the holder main body 12 is set as a lower side as seen from the lid body 14 in the blood filtration unit. That is, the lid body 14 is disposed on the upper side of the holder main body 12. In addition, the blood filtration unit 10 of the present embodiment is used, for example, by being attached to a biochemical analyzer.

### Holder Main Body

The holder main body 12 mainly includes a filtration portion 16 and a blood inflow portion 18. In addition, the material of the holder main body 12 is preferably plastic, for example, transparent or opaque resin such as polymethacrylic acid ester, polyethylene, polypropylene, polyester, nylon, and polycarbonate is used. In the present embodiment, the holder main body 12 is formed of, for example, high impact polystyrene resin.

The filtration portion 16 is positioned above the holder main body 12, formed in a cylindrical shape, and includes a blood introduction portion 16A, a filtration material housing portion 16B, and a microporous film housing portion 16C.

The blood introduction portion 16A is formed in a hollow, substantially conical shape of which the diameter gradually increases from the lower side to the upper side, and a lower end portion of the blood introduction portion 16A communicates with the blood inflow portion 18 to be described below. In addition, a first stage portion 22 is formed at an upper end portion of an inner wall of the blood introduction portion 16A, and a side upper than the first stage portion 22 is formed as the filtration material housing portion 16B.

The filtration material housing portion 16B is formed in a substantially cylindrical shape having a diameter larger than that of the blood introduction portion 16A. As an example of a plurality of the blood filtration materials, a plurality of the glass fiber filter paper sheets 20 are housed inside the filtration material housing portion 16B in a laminated state. In the present embodiment, six sheets of glass fiber filter paper 20 are laminated. In addition, a second stage portion 24 is formed at an upper end portion of an inner wall of the filtration material housing portion 16B, and a side upper than the second stage portion 24 is formed as the microporous film housing portion 16C.

The microporous film housing portion 16C is formed in a substantially cylindrical shape having a diameter larger than that of the filtration material housing portion 16B. As an example of a microporous film, a porous polysulfone film 21 is housed in the microporous film housing portion 16C. The porous polysulfone film 21 is disposed on the second stage portion 24, and the upper surface of the porous polysulfone film 21 comes into contact with the lid body 14. The lower portion of the lid body 14 enters the microporous film housing portion 16C.

The volume of the housing portions such as the filtration material housing portion 16B and the microporous film housing portion 16C is necessarily greater than the total volume of a blood filtration material, to be stored, which has been swollen by absorbing a specimen (whole blood) while being in a dry state. In a case where the volume of the storage portion is smaller than the total volume of the blood filtration material, filtration does not proceed efficiently or hemolysis occurs. The ratio of the volume of the storage portion to the total volume of the dry blood filtration material depends on the degree of swelling of the blood filtration material, but is usually 101% to 400%, preferably 110% to 150%, and more preferably 120% to 140%.

In addition, the space between the blood filtration material and the wall surface of the storage portion is formed such that a flow path which does not pass through the blood filtration material is not formed when whole blood is absorbed. However, there is no problem even in a case where blood cells leak to such a degree that they can be stopped by the microporous film.

Furthermore, the type of blood filtration material is not limited. However, in the blood filtration material of the present embodiment, a so-called volume filtration material such as glass fiber filter paper is used instead of a so-called surface filtration material which traps blood cells only on its surface. That is, in the configuration, blood cells are removed over the entire length in the thickness direction by trapping large blood cell components first and small blood cell components later as the blood cell components penetrate in the thickness direction. A glass fiber filtration paper or a microporous film is preferable and a combination of a glass fiber filter paper and a microporous film is particularly preferable.

The density of glass fiber filter paper is about 0.05 to 0.5 and preferably about 0.07 to 0.35. In addition, glass fiber filter paper having a density of about 0.09 to 0.2 is particularly preferable. Furthermore, glass fiber filter paper having a retained particle diameter of about 0.8 to 9 µm is preferable and glass fiber filter paper having a retained particle diameter of about 1 to 5 µm is particularly preferable.

In a case where the surface of the glass fiber is treated with a hydrophilic polymer through methods described in JP1990-208565A (JP-H02-208565A) and JP1992-208856A (JP-H04-208856A), it is possible to more rapidly perform filtration. In addition, it is also possible to treat the surface of the glass fiber with lectin. A plurality of glass fiber filter paper sheets can be laminated and used.

A microporous film of which the surface is hydrophilized and has a blood cell separation ability specifically separates blood cells and plasma from whole blood without substantially causing hemolysis to such a degree of affecting analysis values.

A pore diameter of the microporous film is a pore diameter smaller than the retained particle diameter of an aggregate of glass fiber filter paper or ultrafine fiber and is suitably greater than or equal to 0.2 µm. In addition, microporous films having a pore diameter of about 0.3 to 5 µm are preferable, and microporous films having a pore diameter of about 0.5 to 3 µm are more preferable. Furthermore, the void volume of the microporous film is preferably high. Specifically, microporous films having a void volume of about 40% to about 95% are suitable. In addition, microporous films having a void volume of about 50% to about 95% are preferable. Furthermore, microporous films having a void volume of about 70% to about 95% are more preferable. Examples of the microporous films include a polysulfone film and a fluorine-containing polymer film.

The microporous film is preferably a polysulfone film, a cellulose acetate film, or the like. In addition, the microporous film is particularly preferably a polysulfone film. In the blood filtration material, at least one of an aggregate of glass fiber filter paper or ultrafine fiber or a three-dimensional porous body is disposed on a blood supply side and the microporous film is disposed on a suction side. The most preferred material is a laminate obtained by laminating an aggregate of glass fiber filter paper or ultrafine fiber and a polysulfone film in this order from the blood supply side.

The filtration material to be used in the present invention can be integrated by bonding layers to each other with a partially disposed adhesive according to methods disclosed in JP1987-138756A (JP-S62-138756A) to JP1987-138758A (JP-S62-138758A), JP1990-105043A(JP-H02-105043A), JP1991-16651A(JP-H03-16651A), and the like.

The amount of whole blood that can be filtered through the present system is greatly influenced by the volume of spaces existing in the aggregate of the glass fiber filter paper or the ultrafine fiber and the volume of blood cells in whole blood. In a case where the density of the aggregate of the glass fiber filter paper or the ultrafine fiber is high (in a case where a particle-holding pore diameter is small), blood cells are trapped in the vicinity of the surface of the aggregate of the glass fiber filter paper or the ultrafine fiber. Therefore, in many cases, a space in the aggregate of the glass fiber filter paper or the ultrafine fiber in a region significantly shallow from the surface is blocked. Accordingly, filtration no longer proceeds, and as a result, the amount of plasma or serum that can be filtered (collected) is reduced. At this time, in a case where a pressure is applied under stronger conditions to increase the amount of liquid to be collected, destruction of blood cells, that is, hemolysis occurs. In other words, it becomes a process similar to surface filtration, and a space volume utilization efficiency of filter paper is low.

On the other hand, in a case where the density of the aggregate of the glass fiber filter paper or the ultrafine fiber is lowered, blood cells penetrate to a deep portion (a region close to an outlet) and the space through which plasma or serum can pass increases. Therefore, the space volume of the entirety of the filter paper is effectively used and the amount of plasma or serum to be collected also increases.

The water permeation rate is effective as an index corresponding to the space volume or the blood filtration amount. The water permeation rate is represented by the filtration amount per unit area in terms of speed in a case where a certain area of an aggregate of glass fiber filter paper or ultrafine fiber in a filtration unit of which an inlet and an outlet are pressed so as to be able to connect to a tube is closed and held, a certain amount of water and is added thereto, and a certain amount of pressure is added or reduced.

As a specific example, an aggregate of glass fiber filter paper or ultrafine fiber having a diameter of 20 mm is set in a filtration unit, a 100 ml injection cylinder is placed thereon, 60 ml of water is poured and allowed to naturally flow down. The amount of water passed through the aggregate of glass fiber filter paper or ultrafine fiber for 30 seconds between 10 seconds and 40 seconds after starting the pouring is set as a water permeation amount, and the water permeation rate per unit area is calculated therefrom.

Filter paper having a water permeation rate of about 1.0 to 1.3 ml/sec is particularly suitable for filtering blood, and examples thereof include GF/D of WATT MANN CO., LTD. and GA-100 and GA-200 of Toyo Roshi Kaisha, Ltd. Furthermore, low-density filter paper (with a density of about 0.03) can be prepared by re-dispersing commercially available glass fiber filter paper in hot water and re-making paper on a nylon net, and this exhibits favorable blood filtration characteristics.

The thickness of glass fiber filter paper is determined from the amount of plasma or serum to be collected and the density (void volume) and the area of the glass fiber filter paper. The required amount of plasma or serum in a case of analyzing a plurality of items using a dry analysis element is 100 to 500 µl, the density of the glass fiber filter paper is practically about 0.07 to 0.2, and the area is practically about 1 to 5 cm². In this case, the thickness of the glass fiber filter paper is about 1 to 10 mm and preferably about 2 to 8 mm. A plurality of glass fiber filter paper sheets, for example, about 2 to 10 sheets, preferably about 2 to 6 sheets can be laminated to obtain the above-described thickness.

The volume of an aggregate of ultrafine fiber can be set to a predetermined size according to the amount of a specimen to be supplied or the required amount of filtered plasma in a place of a biochemical examination. For example, a circular aggregate having a diameter of about 20 mm and a thickness of about 2 to 10 mm can be used.

The thickness of a microporous film may be about 0.05 to 0.5 mm and particularly about 0.1 to 0.3 mm. In general, a microporous film may be used. However, it is also possible to use a plurality of microporous films as necessary.

The blood inflow portion 18 is formed in a substantially tubular shape having a diameter smaller than that of the filtration portion 16 while setting the axial direction (vertical direction) as a longitudinal direction. In addition, an opening 18A is formed at a lower end portion of the blood inflow portion 18, and blood flows into the blood inflow portion 18 from the opening 18A. In a case of blood filtration, a suction nozzle (not shown in the drawing) is installed in the lower end portion of the blood inflow portion 18.

The nozzle for sucking blood may be integral with the holder main body 12 or may be a separate body. In a case where the nozzle is a separate body as in the present embodiment, a connection portion, with the blood inflow portion 18, which is fixed to the holder main body 12 may be a closed structure, and connection means may be adhesion, fusion welding, screwing, fitting, screw-locking, and the like.

### Lid Body

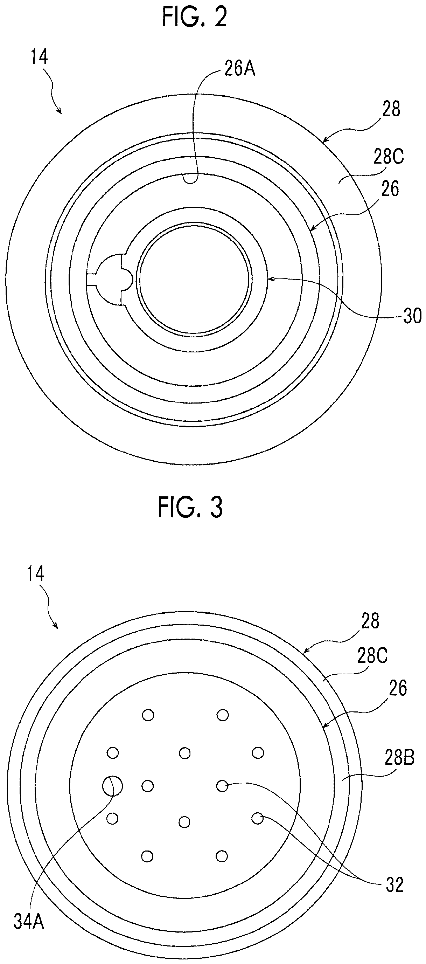

As shown in Figs. 1 and 2, the lid body 14 is attached to an upper side of the holder main body 12 and mainly includes a protection sheath portion 26, a joint portion 28, a storage portion 30, a filtrate flow path portion 34, and an outflow portion 36.

The protection sheath portion 26 is formed in a bottomed cylindrical shape with its upper end side opened while setting the vertical direction as an axial direction, and is formed substantially coaxially with the filtration portion 16. In addition, a pump unit of a biochemical analyzer (not shown in the drawing) is connected to an opening portion 26A formed at an upper end portion of the protection sheath portion 26. The blood filtration unit 10 is configured such that, by operating the pump unit, the internal pressure of the blood filtration unit becomes negative and blood is sucked from a suction nozzle (not shown in the drawing) installed in the lower end portion of the blood inflow portion 18.

Here, as shown in Fig. 3, a plurality of protrusions 32 are formed on the bottom surface of the protection sheath portion 26. For example, twelve protrusions 32 are formed in the present embodiment. As shown in Fig. 1, each of the protrusions 32 protrudes downward from the bottom surface of the protection sheath portion 26, and is formed in a substantially hemispherical shape. The protrusions 32 suppress close attachment of the porous polysulfone film 21 to the bottom surface of the protection sheath portion 26.

The joint portion 28 is provided on the outer circumferential side of the lower end portion of the protection sheath portion 26. The joint portion 28 is provided with an extension portion 28A extending from the lower end portion of the protection sheath portion 26 to the outer circumferential side, and the lower surface of the extension portion 28A abuts on the upper surface of the porous polysulfone film 21. A vertical wall portion 28B extends upward from an end portion of the extension portion 28A on the outer circumferential side. The outer diameter of the vertical wall portion 28B is substantially the same as the inner diameter of the microporous film housing portion 16C of the holder main body 12. The vertical wall portion 28B is fitted inside the microporous film housing portion 16C.

A flange portion 28C extends from the upper end portion of the vertical wall portion 28B to the outer circumferential side. The lid body 14 is attached to the holder main body 12 using the flange portion 28C joined to the upper end surface of the filtration portion 16 through ultrasound welding. The method for attaching the lid body 14 to the holder main body is not limited to the ultrasound welding, and joining using an adhesive may be employed. In addition, the lid body may be joined using mechanical fastening members such as bolts.

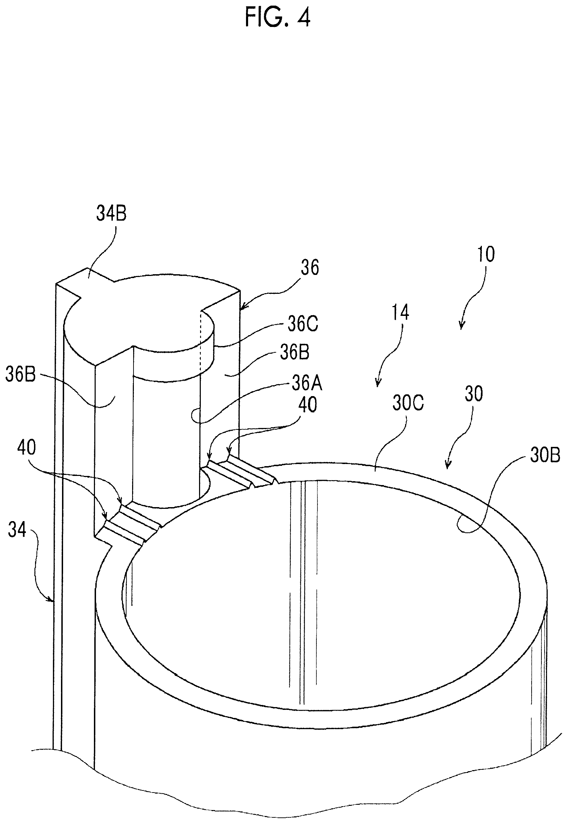

The storage portion 30 is provided on the inner circumferential side of the protection sheath portion 26. The storage portion 30 has a smaller diameter than that of the protection sheath portion 26 and is formed in a bottomed cylindrical shape with one end (upper end) opened while setting the vertical direction as an axial direction. The bottom surface of the storage portion 30 is formed by a part of the bottom surface of the protection sheath portion 26. In addition, the inner wall on the lower side of the storage portion 30 is formed as a tapered surface 30A of which the diameter decreases toward the lower side. Furthermore, an opening 30B formed at the upper end portion of the storage portion 30 is connected to the outflow portion 36 to be described below, and a filtrate which has been filtered by the filtration portion 16 is stored in the storage portion 30. As shown in Fig. 4, a plurality of protruding portions 40 as guide portions are formed on the opening end surface 30C of the storage portion 30. Details of the protruding portions 40 will be described below.

The volume of the storage portion 30 is about 100 to 900 µl and generally about 200 to 600 µl in a case of preparing a sample for dry analysis. In addition, the depth of the storage portion 30 is about 3 to 12 mm and the lateral width (diameter or side) is about 5 to 11 mm.

The filtrate flow path portion 34 is provided between the protection sheath portion 26 and the storage portion 30. The filtrate flow path portion 34 is formed in the vertical direction along the outer circumference (outer wall) of the storage portion 30 and includes a flow path 34A, through which a filtrate flows, between a part of the inner wall of the protection sheath portion 26 and a part of the outer wall of the storage portion 30. The lower end portion of the flow path 34A is open onto the bottom surface of the protection sheath portion 26 and communicates with the filtration portion 16 of the holder main body 12. In addition, the upper end portion of the flow path 34A is closed by an upper wall 34B formed above the upper end portion of the storage portion 30, and a space between the upper wall 34B and the upper end portion of the storage portion 30 is set as an outflow port 36A to be described below. In this manner, the blood filtration unit is configured such that the filtrate which has been filtered by the filtration portion 16 is sucked up into the flow path 34A and flows out from the outflow port 36A.

The outflow portion 36 is formed on the upper side of the filtrate flow path portion 34. As shown in Fig. 4, the outflow portion 36 has a pair of wall portions 36B protruding beyond an upper end (one end) of the storage portion 30 from the upper end portion of the filtrate flow path portion 34, and the outflow port 36A formed between the wall portions 36B. In addition, the outflow port 36A is formed along the opening end surface 30C of the storage portion 30. That is, the outflow port 36A is open onto the opening end surface 30C.

The pair of wall portions 36B are provided on both sides while interposing the outflow port 36A therebetween, and the upper end portions of the pair of wall portions 36B are connected by a hood portion 36C. In addition, the lower end portions of the pair of wall portions 36B are connected to the opening end surface 30C of the storage portion 30, and the wall portions 36B are continuously formed upward from the opening end surface 30C. For this reason, the rim of the outflow port 36A is formed by the pair of wall portions 36B, the hood portion 36C, and the opening end surface 30C of the storage portion 30, and the outflow port 36A is formed in a substantially rectangular shape as seen from the front (in a direction opposite to the wall portions 36B) while setting the vertical direction as a longitudinal direction.

The lower edge of the outflow port 36A is positioned 0.5 to 5 mm above a designed liquid level of the storage portion 30, and is usually positioned 1 to 2 mm above the designed liquid level of the storage portion 30. Here, the amount of filtrate varies depending on a hematocrit value of blood. The designed liquid level is a liquid level when blood with a hematocrit value of 20% to 60% is filtered.

The hood portion 36C is formed so as to protrude from the upper wall 34B of the filtrate flow path portion 34 to the inside of the storage portion 30 and constitutes a part of the upper wall 34B of the filtrate flow path portion 34. In addition, the hood portion 36C protrudes from the pair of wall portions 36B to the inside of the storage portion 30 and suppresses liquid droplets of a filtrate flowing out from the outflow port 36A from going around the upper wall 34B.

### Guide Portion

The plurality of protruding portions 40 as guide portions are formed on the opening end surface 30C of the storage portion 30. Each of the protruding portions 40 extends from the wall portions 36B toward the opening 30B of the storage portion 30. In this embodiment, for example, two protruding portions 40 extend from one of the wall portions 36B and two protruding portions extend from the other one of the wall portions 36B. That is, a total of four protruding portions 40 extend. In addition, the four protruding portions 40 extend parallel to each other from the wall portions 36B to the opening 30B.

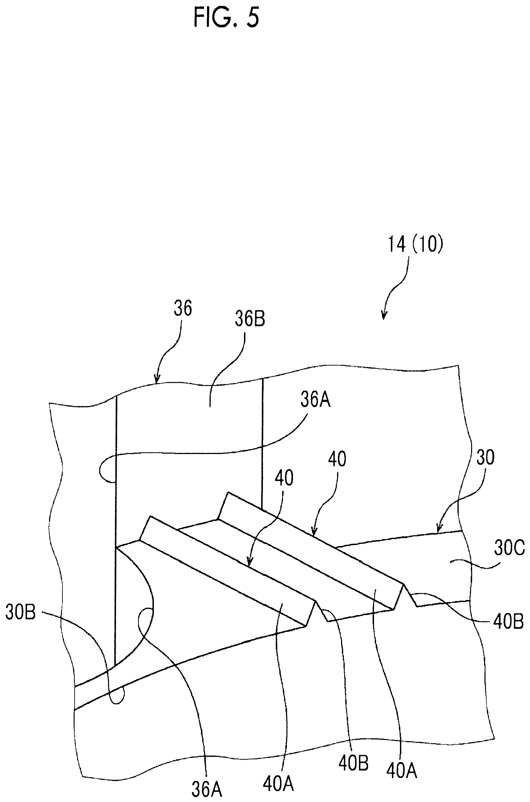

As shown in Fig. 5, the protruding portions 40 have a triangular cross section cut in a direction orthogonal to the extending direction and include a first inclined surface 40A on a side of the outflow port 36A and a second inclined surface 40B on a side opposite to the outflow port 36A. Here, the heights of the protruding portions 40 with respect to the opening end surface 30C are set to 1 µm to 2 mm. In the present embodiment, the heights are set, for example, to 0.1 mm. In addition, each of the protruding portions 40 has an isosceles triangle shape of which the length from the lower end portion of the first inclined surface 40A to the lower end portion of the second inclined surface 40B is 0.12 mm as seen from the extending direction.

Furthermore, the interval between a protruding portion 40 which is close to the outflow port 36A out of the protruding portions 40 extending from one wall portion 36B and a protruding portion 40 which is close to the outflow port 36A out of the protruding portions 40 extending from the other wall portion 36B is set to 1.8 mm. Furthermore, the interval (pitch) between adjacent protruding portions 40 formed on one wall portion 36B is set to 5 µm to 1 mm. In the present embodiment, the interval is set, for example, to 0.4 mm. The interval referred to herein between the protruding portions 40 is a distance of a connection portion between the first inclined surface 40A and the second inclined surface 40B in the protruding portions 40. That is, the interval is a distance between apex portions of the protruding portions 40.

### Action and Effect

Next, actions and effects of the present embodiment will be described.

In the present embodiment, in Fig. 1, blood flows into the blood inflow portion 18 by inserting a suction nozzle (not shown in the drawing) installed in the blood inflow portion 18 into a blood collection tube and operating a pump unit (not shown in the drawing) connected to the opening portion 26A of the protection sheath portion 26. The blood flowing into the blood inflow portion 18 passes through the blood introduction portion 16A of the filtration portion 16 and is filtered while passing through the glass fiber filter paper 20 and the porous polysulfone film 21. Then, the filtrate which has been filtered flows through the flow path 34A of the filtrate flow path portion 34 and flows from the outflow port 36A to the inside of the storage portion 30. In this manner, it is possible to store plasma or serum as a filtrate in the storage portion 30.

In the present embodiment, it is possible to suppress wet-spreading of a filtrate by providing the protruding portions 40 on the opening end surface 30C of the storage portion 30 as shown in Figs. 4 and 5. This effect will be described below while being compared with a comparative example.

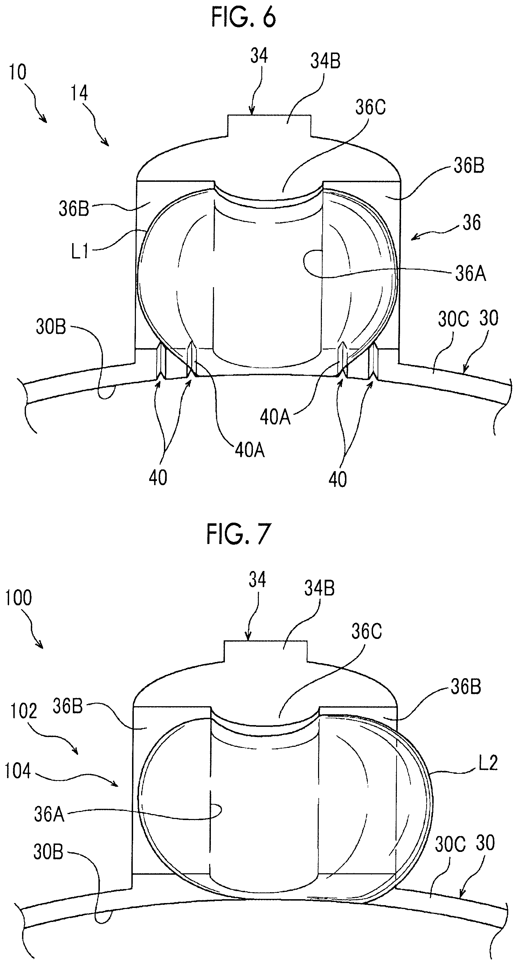

Fig. 6 shows an enlarged view of the periphery of the outflow portion 36 in the lid body 14 of the blood filtration unit 10 of the present embodiment and Fig. 7 shows an enlarged view of the periphery of an outflow portion 104 of a lid body 102 of a blood filtration unit 100 of the comparative example. Here, the blood filtration unit 100 of the comparative example is different from the present embodiment in that it does not include the protruding portions 40 as guide portions, and other configurations are the same as those of the present embodiment. Therefore, detailed description will not be repeated.

As shown in Fig. 7, a filtrate sucked up to the outflow portion 104 from the filtrate flow path portion 34 becomes a liquid droplet L2 in the vicinity of the outflow port 36A. Here, in the configuration in which the protruding portions 40 are not formed on the opening end surface 30C of the storage portion 30, the liquid droplet L2 of the filtrate flowing out from the outflow port 36A increases while coming into contact with the hood portion 36C and the opening end surface 30C. In some cases, at least a part of the liquid droplet L2 flows down to the outside of the storage portion 30 as the liquid droplet L2 wet-spreads in the circumferential direction of the storage portion 30 along the opening end surface 30C.

On the other hand, as shown in Fig. 6, in the structure of the present embodiment, a liquid droplet L1 tends to wet-spread while coming into contact with the hood portion 36C and the opening end surface 30C. However, the liquid droplet L1 comes into contact with a first inclined surface 40A of a protruding portion 40 extending from one wall portion 36B and a first inclined surface 40A of a protruding portion 40 extending from the other wall portion 36B, thereby suppressing the wet-spreading of the liquid droplet L1. Accordingly, it is possible to suppress the liquid droplet L1 from flowing down to the outer circumferential side of the storage portion 30.

In addition, in the present embodiment, it is possible to make a rapid angle variation between the first inclined surface 40A and the second inclined surface 40B by making the cross sections of the protruding portions 40 to have a triangular shape. Accordingly, this makes it difficult for the liquid droplet L1 of a filtrate to go around (climb over) compared to a case of using protruding portions of which the apex portions are made to be curved surfaces, and therefore, it is possible to effectively suppress the wetting.

Furthermore, in the present embodiment, the pair of protruding portions 40 formed to interpose the outflow port 36A therebetween extend in parallel with each other. Therefore, it is possible to make the liquid droplet L1 of the filtrate reliably come into contact with the protruding portions 40 on both sides of the outflow port 36A and to effectively suppress the wetting of the filtrate.

Furthermore, in the present embodiment, it is possible to effectively suppress the wetting of the filtrate by setting the heights of the protruding portions 40 with respect to the opening end surface 30C to be 1 µm to 2 mm. That is, in a case where the heights of the protruding portions 40 with respect to the opening end surface 30C is lower than 1 µm, the function as the protruding portions 40 cannot be sufficiently exerted. In addition, in a case where the heights of the protruding portions 40 with respect to the opening end surface 30C is set to be higher than 2 mm, a holding volume of liquid droplets is increased, and therefore, the filtrate easily flows to the outside of the storage portion 30.

In addition, in the present embodiment, there are two protruding portions 40 formed to interpose the outflow port 36A therebetween. Therefore, even in a case where the liquid droplet L1 climbs over one protruding portion 40, it is possible to suppress wet-spreading of the liquid droplet L1 at the second protruding portion 40. That is, it is possible to suppress the wet-spreading of the filtrate effectively compared to a configuration in which one protruding portion 40 is respectively provided in one wall portion 36B and the other wall portion 36B.

Furthermore, in the present embodiment, it is possible to effectively suppress the wet-spreading of the filtrate by setting the pitch between adjacent protruding portions 40 to be 5 µm to 1 mm. That is, in a case where the pitch between the adjacent protruding portions is less than 5 µm, it is difficult to be processed and to obtain desired dimensional accuracy. In addition, in a case where the pitch is greater than 1 mm, the wet-spreading amount until the liquid droplet L1 comes into contact with the protruding portions 40 increases.

Furthermore, in the present embodiment, the blood filtration material is configured to include the glass fiber filter paper 20 and the porous polysulfone film 21. Therefore, it is possible to suppress leakage or elution of blood cell components compared to a configuration in which only one of the glass fiber filter paper 20 and the porous polysulfone film 21 is provided. In addition, it is possible to effectively perform the filtration compared to a case where a blood filtration material having a void volume lower than 40% is used, by setting the void volume of the blood filtration material to 40% to 95%.

### Second Embodiment

Next, a second embodiment of the present invention will be described with reference to the drawings. Components having the same configuration as that of the first embodiment are given of the same reference numerals, and the detailed description thereof will not be repeated.

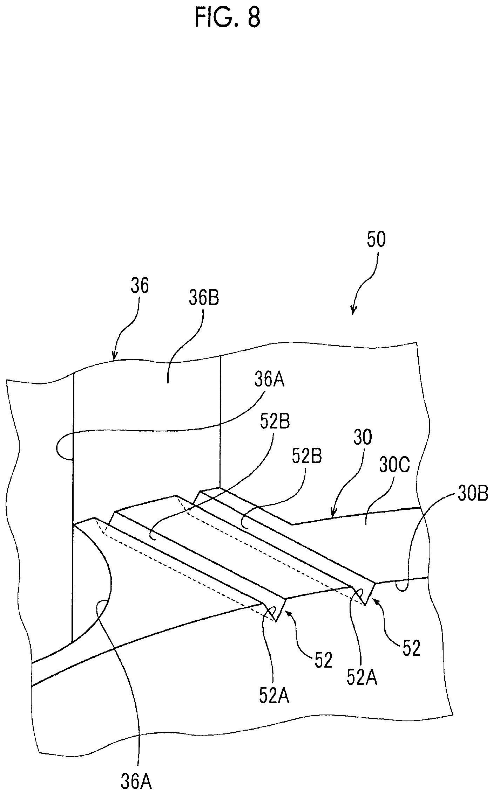

A blood filtration unit 50 of the present embodiment has the same configuration as that of the first embodiment except for the guide portions formed on the opening end surface 30C of the storage portion 30. For this reason, only the guide portions will be described. As shown in Fig. 8, a plurality of groove portions 52 as guide portions are formed on the opening end surface 30C of the storage portion 30.

Each of the groove portions 52 extends from the wall portions 36B toward the opening 30B of the storage portion 30. In this embodiment, for example, two groove portions 52 extend from one of the wall portions 36B and two groove portions extend from the other one of the wall portions 36B. That is, a total of four groove portions 52 extend. In addition, the four groove portions 52 extend parallel to each other from the wall portions 36B to the opening 30B. Although only two groove portions 52 formed in one wall portion 36B are illustrated in Fig. 8, there are also two groove portions 52 formed in the other wall portion 36B while interposing the outflow port 36A therebetween.

The groove portions 52 have a shape cut out into a substantially triangle as seen from a direction orthogonal to the extending direction and include a first groove wall 52A on a side of the outflow port 36A and a second groove wall 52B on a side opposite to the outflow port 36A. Here, the depths of the groove portions 52 with respect to the opening end surface 30C are set to 1 µm to 2 mm. In the present embodiment, the depths are set, for example, to 0.1 mm. In addition, the groove width of each of the groove portions 52 is set to 0.12 mm.

Furthermore, the interval between a groove portion 52 which is close to the outflow port 36A out of the groove portions 52 extending from one wall portion 36B and a groove portion 52 which is close to the outflow port 36A out of the groove portions 52 extending from the other wall portion 36B is set to 1.8 mm. Furthermore, the interval (pitch) between adjacent groove portions 52 formed on one wall portion 36B is set to 5 µm to 1 mm. In the present embodiment, the interval is set, for example, to 0.4 mm. The interval referred to herein between the groove portions 52 is a distance of a connection portion between the first groove wall 52A and the second groove wall 52B in the groove portions 52. That is, the interval is a distance between groove bottoms of the groove portions 52.

### Action and Effect

Next, actions and effects of the present embodiment will be described.

In the present embodiment, it is possible to suppress wet-spreading of a filtrate since liquid droplets of the filtrate stop at an edge between the first groove wall 52A and the opening end surface 30C in the groove portions 52.

In addition, since the groove portions 52 are formed to be parallel to each other, it is possible to make the liquid droplets of the filtrate reliably follow edges of the groove portions 52 on both sides of the outflow port 36A and to effectively suppress the wet-spreading of the filtrate.

Furthermore, it is possible to effectively suppress the wet-spreading of the filtrate by setting the depths of the groove portions 52 with respect to the opening end surface 30C to the above-described range. That is, in a case where the depths of the groove portions 52 with respect to the opening end surface 30C are shallower than 1 µm, the function as the groove portions 52 cannot be sufficiently exerted. In addition, in a case where the depths of the groove portions 52 with respect to the opening end surface 30C are made deeper than 2 mm, the moldability in a case of molding performed through injection molding sometimes decreases.

Furthermore, since a plurality of groove portions 52 are formed, even in a case where the liquid droplets pass through one groove portion 52, it is possible to suppress the wet-spreading of the liquid droplets at a second groove portion 52. That is, it is possible to suppress the wet-spreading of the filtrate effectively compared to a configuration in which one groove portion 52 is respectively provided in one wall portion 36B and the other wall portion 36B.

In addition, it is possible to effectively suppress the wet-spreading of the filtrate by setting the pitches of adjacent groove portions 52 to the above-described range. That is, in a case where the pitch between the adjacent groove portions 52 is less than 5 µm, it is difficult to be processed and to obtain desired dimensional accuracy. In addition, in a case where the pitch is greater than 1 mm, the wet-spreading amount until the liquid droplets come into contact with the edge of the groove portions 52 increases.

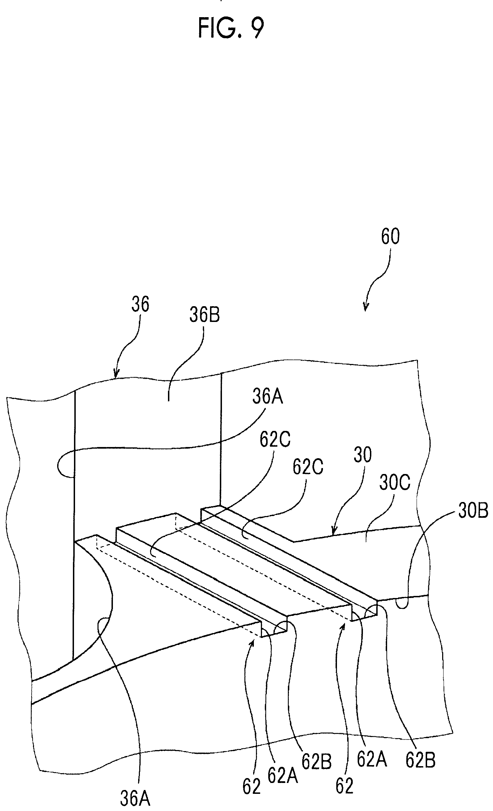

The shapes of the groove portions 52 are not limited to the present embodiment, and other shapes may be employed. For example, a rectangular groove portion 62 may be formed as in a modification example shown in Fig. 9.

### Modification Example

As shown in Fig. 9, a plurality of groove portions 62 are formed on the opening end surface 30C of the storage portion 30 in a blood filtration unit 60 according to the present modification example. Each of the groove portions 62 extends from the wall portions 36B toward the opening 30B of the storage portion 30. In this embodiment, for example, two groove portions 62 extend from one of the wall portions 36B and two groove portions 62 extend from the other one of the wall portions 36B. That is, a total of four groove portions 62 extend. In addition, the four groove portions 62 extend parallel to each other from the wall portions 36B to the opening 30B. Although only two groove portions 62 formed in one wall portion 36B are illustrated in Fig. 9, there are also two groove portions 62 formed in the other wall portion 36B while interposing the outflow port 36A therebetween.

The groove portions 62 have a shape cut out into a rectangle as seen from a direction orthogonal to the extending direction and include a first groove wall 62A on a side of the outflow port 36A, a second groove wall 62C on a side opposite to the outflow port 36A, and a groove bottom wall 62B connecting the first groove wall 62A with the second groove wall 62C.

According to the present modification example, it is possible to reduce an angle between the opening end surface 30C and the first groove wall 62A and to effectively suppress the wet-spreading of the filtrate, compared to the case where the groove portions 62 have a shape cut out into a triangle.

Although the embodiments and the modification example of the present invention have been described above, the present invention is not limited to the above-described embodiments and modification example, and various modifications can be made within the scope of the invention as defined by the claims. For example, in the above-described first embodiment, the protruding portions 40 are formed in a triangular shape. However, the present invention is not limited thereto, and other shapes may be employed. In addition, the second inclined surface 40B of the protruding portions 40 may be formed perpendicularly to the opening end surface 30C. In this case, the angle between the first inclined surface 40A and the second inclined surface 40B becomes sharper, and it is possible to effectively suppress the wet-spreading of the liquid droplets.

In addition, both the protruding portions 40 and the groove portions 52 may be formed on the opening end surface 30C by combining the first embodiment and the second embodiment.

## Claims

1. A blood filtration unit (10, 50, 60) comprising:
a filtration portion (16) in which a blood filtration material (20) is housed;
a blood inflow portion (18) through which blood flows into the filtration portion (16);
a storage portion (30) which has a tubular shape with one end opened and in which a filtrate filtered by the filtration portion (16) is stored;
a filtrate flow path portion (34) which is formed along the outer periphery of the storage portion and includes a flow path (34A) through which the filtrate flows;
an outflow portion (36) in which an outflow port (36A) is formed between a pair of wall portions (36B) protruding beyond the one end of the storage portion (30) from an end portion of the filtrate flow path portion (34)along an opening end surface (30C) of the one end of the storage portion (30), wherein
the outflow port (36A) is open onto the opening end surface (30C); and
**characterized by**
a guide portion which is formed on the opening end surface of the storage portion and includes at least one of a protruding portion (40) or a groove portion (52, 62) extending from the pair of wall portions toward the opening (30B).

2. The blood filtration unit (10) according to claim 1,
wherein the guide portion includes the protruding portion (40).

3. The blood filtration unit (10) according to claim 2,
wherein the protruding portion (40) has a triangular cross section cut in a direction orthogonal to an extending direction.

4. The blood filtration unit (10) according to claim 2 or 3,
wherein the protruding portions (40) extend parallel to each other from one of the wall portions (36B) and the other wall portion while interposing the outflow port (36A) therebetween.

5. The blood filtration unit (10) according to any one of claims 2 to 4,
wherein a height of the protruding portion (40) with respect to the opening end surface (30C) is 1 µm to 2 mm.

6. The blood filtration unit (10) according to any one of claims 2 to 5,
wherein a plurality of the protruding portions (40) extend from one of the wall portions (36B) and the other wall portion while interposing the outflow port (36A) therebetween.

7. The blood filtration unit (10) according to claim 6,
wherein an interval between the protruding portions (40) adjacent to each other on the one wall portion side and an interval between the protruding portions (40) adjacent to each other on the other wall portion side are 5 µm to 1 mm.

8. The blood filtration unit (50, 60) according to claim 1,
wherein the guide portion includes the groove portion (52, 62).

9. The blood filtration unit (60) according to claim 8,
wherein the groove portion (62) has a shape cut out into a rectangle as seen from an extending direction.

10. The blood filtration unit (50, 60) according to claim 8 or 9,
wherein the groove portions (52, 62) extend parallel to each other from one of the wall portions (36B) and the other wall portion while interposing the outflow port (36A) therebetween.

11. The blood filtration unit (50, 60) according to any one of claims 8 to 10,
wherein a depth of the groove portion (52, 62) with respect to the opening end surface (30C) is 1 µm to 2 mm.

12. The blood filtration unit (50, 60) according to any one of claims 8 to 11,
wherein a plurality of the groove portions (52, 62) extend from one of the wall portions (36B) and the other wall portion while interposing the outflow port (36A) therebetween.

13. The blood filtration unit (50, 60) according to claim 12,
wherein an interval between the groove portions (52, 62) adjacent to each other on the one wall portion side and an interval between the groove portions adjacent to each other on the other wall portion side are 5 µm to 1 mm.

14. The blood filtration unit (10, 50, 60) according to any one of claims 1 to 13,
wherein the blood filtration material includes glass fiber filter paper (20) and a microporous film.

15. The blood filtration unit according to any one of claims 1 to 14,
wherein the blood filtration material is formed of a material of which a void volume is 40% to 95%.

## Patentansprüche

1. Blutfiltriereinheit (10, 50, 60), umfassend:
einen Filtrierabschnitt (16), in dem Blutfiltriermaterial (20) aufgenommen ist;
einen Bluteinströmabschnitt (18), durch den hindurch Blut in den Filtrierabschnitt (16) strömt;
einen Speicherabschnitt (30) mit rohrförmiger Gestalt und mit einem offenen Ende, in dem ein von dem Filtrierabschnitt (16) filtriertes Filtrat gespeichert wird;
einen Filtrat-Strömungswegabschnitt (34), der entlang dem Außenumfang des Speicherabschnitts ausgebildet ist und einen Strömungsweg (34A) enthält, durch den hindurch das Filtrat strömt;
einen Ausströmabschnitt (36), in welchem eine Ausströmöffnung (36A) zwischen einem Paar von Wandabschnitten (36B) ausgebildet ist, welche über das eine Ende des Speicherabschnitts (30) von einem Endabschnitts des Filtrat-Strömungswegabschnitts (34) entlang einer Öffnungsendfläche (30C) des einen Endes des Speicherabschnitts (30) vorstehen;
wobei die Ausströmöffnung (36A) zu der Öffnungsendfläche (30C) offen ist,
**gekennzeichnet durch** einen Führungsabschnitt, der an der Öffnungsendfläche des Speicherabschnitts ausgebildet ist und mindestens einen vorstehenden Abschnitt (40) und/oder einen Nutenabschnitt (52, 62) enthält, der sich von dem Paar Wandabschnitten in Richtung der Öffnung (30B) erstreckt.

2. Blutfiltriereinheit (10) nach Anspruch 1,
bei der der Führungsabschnitt den vorstehenden Abschnitt (40) enthält.

3. Blutfiltriereinheit (10) nach Anspruch 2,
bei der der vorstehende Abschnitt (40) einen Dreieckquerschnitt aufweist, ausgeschnitten in einer Richtung orthogonal zu einer Erstreckungsrichtung.

4. Blutfiltriereinheit (10) nach Anspruch 2 oder 3,
bei der die vorstehenden Abschnitte (40) sich parallel zueinander von einem der Wandabschnitte (36B) und dem anderen Wandabschnitt erstrecken, wobei sie die Ausströmöffnung (36A) zwischen sich einfassen.

5. Blutfiltriereinheit (10) nach einem der Ansprüche 2 bis 4,
bei der eine Höhe des vorstehenden Abschnitts (40) in Bezug auf die Öffnungsendfläche (30C) 1 µm bis 2 mm beträgt.

6. Blutfiltriereinheit (10) nach einem der Ansprüche 2 bis 5,
bei der mehrere vorstehende Abschnitte (40) sich von einem der Wandabschnitte (36B) und dem anderen Wandabschnitt aus erstrecken, wobei sie die Ausströmöffnung (36A) zwischen sich aufnehmen.

7. Blutfiltriereinheit (10) nach Anspruch 6,
bei der ein Intervall zwischen den vorstehenden Abschnitten (40), die einander an der einen Wandabschnittseite benachbart sind, und ein Intervall zwischen den vorstehenden Abschnitten (40), die einander an der anderen Wandabschnittseite benachbart sind, 5 µm bis 1 mm beträgt.

8. Blutfiltriereinheit (50, 60) nach Anspruch 1,
bei der der Führungsabschnitt einen Nutenabschnitt (52, 62) enthält.

9. Blutfiltriereinheit (60) nach Anspruch 8,
bei der der Nutenabschnitt (62) eine zu einem Rechteck bei Betrachtung in Erstreckungsrichtung ausgeschnittene Gestalt aufweist.

10. Blutfiltriereinheit (50, 60) nach Anspruch 8 oder 9,
bei der die Nutenabschnitte (52, 62) sich parallel zueinander von einem der Wandabschnitte (36B) aus und dem anderen Wandabschnitt erstrecken, wobei sie die Auslassöffnung (36A) zwischen sich aufnehmen.

11. Blutfiltriereinheit (50, 60) nach einem der Ansprüche 8 bis 10,
bei der eine Tiefe des Nutenabschnitts (52, 62) in Bezug auf die Öffnungsendfläche (30C) 1 µm bis 2 mm beträgt.

12. Blutfiltriereinheit (50, 60) nach einem der Ansprüche 8 bis 11,
bei der mehrere der Nutenabschnitte (52, 62) sich von einem der Wandabschnitte (36B) und dem anderen Wandabschnitt aus erstrecken, wobei sie die Ausströmöffnung (36A) zwischen sich aufnehmen.

13. Blutfiltriereinheit (50, 60) nach Anspruch 12,
bei der ein Intervall zwischen den Nutenabschnitten (52, 62), die einander an der einen Wandabschnittseite benachbart sind, und ein Intervall zwischen den Nutenabschnitten, die einander an der anderen Wandabschnittseite benachbart sind, 5 µm bis 1 mm betragen.

14. Blutfiltriereinheit (10, 50, 60) nach einem der Ansprüche 1 bis 13,
bei der das Blutfiltriermaterial Glasfaser-Filterpapier (20) und einen mikroporösen Film enthält.

15. Blutfiltriereinheit nach einem der Ansprüche 1 bis 14,
bei der das Blutfiltriermaterial aus einem Material gebildet ist, welches ein Leerraumvolumen von 40% bis 95% besitzt.

## Revendications

1. Unité de filtration du sang (10, 50, 60), comprenant :
une portion de filtration (16), où une matière de filtration du sang (20) est logée ;
une portion de flux entrant de sang (18) à travers laquelle s'écoule le sang jusque dans la portion de filtration (16) ;
une portion de stockage (30), laquelle présente une forme tubulaire dotée d'une extrémité ouverte et où est stocké un filtrat filtré par la portion de filtration (16) ;
une portion de trajet d'écoulement de filtrat (34), laquelle est formée le long de la périphérie extérieure de la portion de stockage et inclut un trajet d'écoulement (34A) à travers lequel s'écoule le filtrat ;
une portion de flux sortant (36), où un orifice de flux sortant (36A) est formé entre une paire de portions de paroi (36B) faisant saillie au-delà de la une extrémité de la portion de stockage (30) à partir d'une portion d'extrémité du trajet d'écoulement de filtrat (34) le long d'une surface d'extrémité d'ouverture (30C) de la une extrémité de la portion de stockage (30), dans laquelle l'orifice de flux sortant (36A) est ouvert sur la surface d'extrémité d'ouverture (30C), et
**caractérisée par** une portion de guidage, laquelle est formée sur la surface d'extrémité d'ouverture de la portion de stockage et inclut au moins une portion parmi une portion saillante (40) ou une portion de rainure (52, 62) s'étendant à partir de la paire de portions de paroi vers l'ouverture (30B).

2. Unité de filtration du sang (10) selon la revendication 1,
dans laquelle la portion de guidage inclut la portion saillante (40).

3. Unité de filtration du sang (10) selon la revendication 2,
dans laquelle la portion saillante (40) présente une section transversale triangulaire en coupe dans une direction orthogonale à une direction d'extension.

4. Unité de filtration du sang (10) selon la revendication 2 ou 3,
dans laquelle les portions saillantes (40) s'étendent parallèlement entre elles à partir de l'une des portions de paroi (36B) et de l'autre portion de paroi, l'orifice de flux sortant (36A) étant interposé entre celles-ci.

5. Unité de filtration du sang (10) selon l'une quelconque des revendications 2 à 4,
dans laquelle une hauteur de la portion saillante (40) par rapport à la surface d'extrémité d'ouverture (30C) est comprise dans une plage allant de 1 µm à 2 mm.

6. Unité de filtration du sang (10) selon l'une quelconque des revendications 2 à 5,
dans laquelle une pluralité des portions saillantes (40) s'étend à partir de l'une des portions de paroi (36B) et de l'autre portion de paroi, l'orifice de flux sortant (36A) étant interposé entre celles-ci.

7. Unité de filtration du sang (10) selon la revendication 6,
dans laquelle un intervalle entre les portions saillantes (40) adjacentes entre elles sur le un côté de portion de paroi et un intervalle entre les portions saillantes (40) adjacentes entre elles sur l'autre côté de portion de paroi sont compris dans une plage allant de 5 µm à 1 mm.

8. Unité de filtration du sang (50, 60) selon la revendication 1,
dans laquelle la portion de guidage inclut la portion de rainure (52, 62).

9. Unité de filtration du sang (60) selon la revendication 8,
dans laquelle la portion de rainure (62) présente une forme découpée en rectangle lorsque que l'on regarde à partir de la direction d'extension.

10. Unité de filtration du sang (50, 60) selon la revendication 8 ou 9,
dans laquelle les portions de rainure (52, 62) s'étendent parallèlement les unes aux autres à partir de l'une des portions de paroi (36B) et de l'autre portion de paroi, l'orifice de flux sortant (36A) étant interposé entre celles-ci.

11. Unité de filtration du sang (50, 60) selon l'une quelconque des revendications 8 à 10,
dans laquelle une profondeur de la portion de rainure (52, 62) par rapport à la surface d'extrémité d'ouverture (30C) est comprise dans une plage allant de 1 µm à 2 mm.

12. Unité de filtration du sang (50, 60) selon l'une quelconque des revendications 8 à 11,
dans laquelle une pluralité des portions de rainure (52, 62) s'étendent à partir de l'une des portions de paroi (36B) et de l'autre portion de paroi, l'orifice de flux sortant (36A) étant interposé entre celles-ci.

13. Unité de filtration du sang (50, 60) selon la revendication 12,
dans laquelle un intervalle entre les portions de rainure (52, 62) adjacentes entre elles sur le un côté de portion de paroi et un intervalle entre les portions de rainure adjacentes entre elles sur l'autre côté de portion de paroi sont compris dans une plage allant de 5 µm à 1 mm.

14. Unité de filtration du sang (10, 50, 60) selon l'une quelconque des revendications 1 à 13,
dans laquelle la matière de filtration du sang inclut un filtre papier à fibres de verre (20) et un film microporeux.

15. Unité de filtration du sang (10, 50, 60) selon l'une quelconque des revendications 1 à 14,
dans laquelle la matière de filtration du sang est formée d'une matière dont le volume mort est compris dans une plage allant de 40 % à 95 %.
